# EUROPÄISCHE PATENTSCHRIFT

(11) **EP 1 496 853 B1**
(45) Veröffentlichungstag und Bekanntmachung des Hinweises auf die Patenterteilung: **26.04.2006**
(21) Anmeldenummer: 03718738.2
(22) Anmeldetag: 08.04.2003
(51) Int. Cl.: A61Q 17/04, A61Q 19/00, A61Q 19/10, A61K 8/02, A61K 8/73

(54) **STÄRKEHALTIGE KOSMETISCHE TÜCHER**
COSMETIC TISSUES CONTAINING STARCH
LINGETTES COSMETIQUES CONTENANT DE L'AMIDON

(30) Priorität: 11.04.2002 DE 10216511
(43) Veröffentlichungstag der Anmeldung: 19.01.2005
(73) Patentinhaber: Beiersdorf AG, 20245 Hamburg (DE)
(72) Erfinder: DÖRSCHNER, Albrecht, 20146 Hamburg (DE); KÜTHER, Jörg, 25421 Pinneberg (DE)
(86) Internationale Anmeldenummer: PCT/EP2003/003610
(87) Internationale Veröffentlichungsnummer: WO 2003/084497

(56) Entgegenhaltungen:
- DE-U- 20 109 450
- US-B1- 6 248 338

## Beschreibung

Die vorliegende Erfindung betrifft ein wasserunlösliches Substrat, getränkt mit einer kosmetischen Zubereitung enthaltend ein oder mehrere vorgelatinisierte, quervernetzte Stärkederivate.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Damit die Haut ihre biologischen Funktionen im vollen Umfang erfüllen kann, bedarf sie der regelmäßigen Reinigung und Pflege sowie dem Schutz vor UV-Strahlung. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen. Zum Schutz vor der schädlichen UV-Strahlung des Sonnenlichtes sind vielen kosmetischen und dermatologischen Hautpflegeprodukten UV-Lichtschutzfilter zugesetzt.

Auch werden spezielle Sonnenschutzprodukte, deren primäre Aufgabe der Schutz vor UV-Strahung ist, in einer großen Produktvielfalt angeboten.

Die meisten kosmetischen und dermatologischen Zubereitungen liegen in Form von mehr oder weniger viskosen Flüssigkeiten vor. Diese zähflüssigen (Salben, Crémes, Gele) oder dünnflüssigen (Lotionen, Wässer) Zubereitungen werden in der Regel mit den Händen oder Fingern ihren Verpackungen entnommen und auf die Haut aufgetragen.

Eine besondere Anwendungsform kosmetischer und/oder dermatologischer Zubereitungen stellen die Tücher dar. Diese können bereits vom Hersteller mit dem Kosmetikum bzw. Dermatikum getränkt sein und haben dadurch den Vorteil, dass in ihnen die Zubereitung bereits in der richtigen Dosierung vorgegeben ist. Außerdem vermeiden sie den Nachteil von in Flaschen und Tiegeln aufbewahrten Zubereitungen, deren Verpackung zerbrechen und deren Inhalt "auslaufen" kann. Ein weiterer Nachteil von Flaschen und Tiegeln gegenüber Tüchern besteht in dem Umstand, dass deren Inhalt bei der Entnahme durch den Kontakt mit Hand und Fingern mit Mikroorganismen kontaminiert wird.

Nachteilig am Stande der Technik ist jedoch der Umstand, dass es bisher nicht gelungen ist, Tücher mit einer kosmetischen Zubereitung enthaltend Stärke oder deren Derivate industriell herzustellen. Stärke und ihre Derivate erzeugen auf der Haut ein angenehm glattes, samtiges Hautgefühl während und nach der Anwendung entsprechender Reinigungs- und Pflegeprodukte und ist daher ein häufig verwendeter Bestandteil kosmetischer Zubereitungen. Nach dem Stande der Technik ist Stärke beziehungsweise ihre Derivate in kosmetischen Zubereitungen schwer und allenfalls in geringen Mengen löslich. Zahlreiche Versuche, Tücher mit einer Stärke(-derivat) haltigen Zubereitung zu besprühen, führten durch Ausfällungen dieser Verbindungen in den Sprühanlagen, zu Verstopfungen der Sprühdüsen und damit zu ungleichmäßig imprägnierten Tüchern beziehungsweise funktionsuntüchtigen Imprägnierungs- und Sprühanlagen.

Eine weitere Methode zur Herstellung von getränkten Tüchern, besteht in dem Eintauchen des Tuches in die kosmetische Zubereitung. Herkömmliche Stärke(-derivat) haltige Zubereitungen weisen jedoch eine zu hohe Viskosität auf, als dass sich Tücher durch Eintauchen in diese Zubereitungen mit diesen gleichmäßig und mit produktrelevanten Mengen der Lösungen tränken ließen.

Es war daher die Aufgabe der vorliegenden Erfindung, die Mängel des Standes der Technik zu beseitigen und Stärke(-derivat) haltige kosmetische Tücher zu entwickeln, die sich mit den herkömmlichen Methoden der Herstellung solcher Tücher in industriellem Maßstab herstellen lassen.

Überraschend gelöst wird die Aufgabe durch ein wasserunlösliches Substrat, imprägniert mit einer kosmetischen Zubereitung enthaltend ein oder mehrere vorgelatinisierte, quervernetzte Stärkederivate.

Die erfindungsgemäßen Produkte zeichnen sich durch ein außergewöhnlich angenehm glattes und samtiges Hautgefühl während und nach der Anwendung aus. Aufgrund ihrer physikalisch-chemischen Eigenschaften (Löslichkeit, Rheologie), lassen sich die erfindungsgemäßen kosmetischen Zubereitungen überraschend problemlos sowohl auf die erfindungsgemäßen Substrate aufsprühen als auch in einem Tauchbad benetzen.

Zwar beschreibt die US 6248338 wässrige Tensidsysteme mit vorgelatinisierten, quervernetzten Stärkederivaten, doch konnte diese Schrift nicht den Weg zur vorliegenden Erfindung weisen.

Erfindungsgemäß bevorzugt ist der Einsatz von Tüchern als wasserunlösliches Substrat. Die erfindungsgemäß vorteilhaften Tücher können glatt oder auch oberflächenstrukturiert sein. Es können geschlossenen oder gelochte Tücher sowie Netze verwendet werden. Erfindungsgemäß bevorzugt sind oberflächenstrukturierte Tücher. Neben Tüchern können erfindungsgemäß vorteilhaft auch Polster, Kissen und Bäusche verwendet werden.

Erfindungsgemäß bevorzugt werden in Kombination mit den erfindungsgemäßen Zubereitungen "trockene" Substrate eingesetzt, welche aus Vlies (nicht gewebtes, "nonwoven" Material) bestehen, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Substrat Verwendung finden soll.

Werden geprägte oder gelochte Vliese verwendet, so erleichtern große Kavitäten an der Vliesoberfläche und im Vlies die Aufnahme von Schmutz und Verunreinigungen, wenn mit dem getränkten Substrat über die Haut gefahren wird. Die Reinigungswirkung kann gegenüber ungeprägten Substraten um ein Vielfaches gesteigert werden.

Es hat sich als vorteilhaft herausgestellt für das Substrat, wenn dieses ein Gewicht von 30 bis 120 g/m², vorzugsweise von 40 bis 80 g/m² und insbesondere bevorzugt 45 bis 60 g/m², hat (gemessen bei 20 °C ± 2 °C und bei einer Feuchtigkeit der Raumluft von 65 % ± 5 % für 24 Stunden).

Die Dicke eines erfindungsgemäßen Vlieses beträgt 0,3 bis 5 mm, vorzugsweise 0,4 mm bis 2 mm, insbesondere 0,6 mm bis 0,9 mm.

Als Ausgangsmaterialien für das wasserunlösliche Substrat können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polytactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform eines Vlieses bestehen die Fasern aus einer Mischung aus und 60 bis 80 % Viskose mit 40 bis 20 % PET insbesondere 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Substrates eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Ferner weisen die zur Bildung des Substrates eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Substrate im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Substrate beträgt vorzugsweise

| | | |
|---|---|---|
| im trockenen Zustand | Maschinenrichtung | 15 % bis 100 %, bevorzugt |
| | | 20 % und 50 % |
| | Querrichtung | 40 % bis 120 %, bevorzugt |
| | | 50 % und 85 % |
| im getränkten Zustand | Maschinenrichtung | 15 % bis 100 %, bevorzugt |
| | | 20 % und 40 % |
| | Querrichtung | 40 % bis 120 %, bevorzugt |
| | | 50 % und 85 % |

Erfindungsgemäß vorteilhaft sind sowohl feucht anmutende als auch trocken anmutende mit kosmetischer Zubereitung getränkte Substrate.

Es ist für die erfindungsgemäßen Substrate, welche nach der Tränkung nicht getrocknet werden erfindungsgemäß vorteilhaft, wenn das Gewichtsverhältnis von Tuch zu kosmetischer Zubereitung 0,7:1 bis zu 1:4 beträgt. Auch größere Mengen an kosmetischer Zubereitung sind erfindungsgemäß vorteilhaft. Der Imprägnierungsgrad des Substrates beträgt also vorteilhafter Weise ≥ 100 Gewichts-%.

Für erfindungsgemäße Substrate, welche nach der Tränkung getrocknet werden ist ein Gewichtsverhältnis von 1:1 bis zu 1:4 nach der Tränkung und vor der Trocknung erfindungsgemäß besonders vorteilhaft. Auch bei diesen Substraten sind größere Mengen an kosmetischer Zubereitung erfindungsgemäß vorteilhaft. Die Menge an verdampfbaren Bestandteilen in der kosmetischen Zubereitung wird aufgrund der sich anschließenden Trocknung eher gering gehalten, d.h. es werden Zubereitungen mit höherer Wirkstoffkonzentration eingesetzt. Der Imprägnierungsgrad des Substrates beträgt vorteilhafter Weise ≥ 150 Gewichts-% und kleiner / gleich 300 Gewichts-%.

Es ist vorteilhaft im Sinne der vorliegenden Erfindung, wenn die erfindungsgemäße kosmetische Zubereitung mit der das wasserunlösliche Substrat getränkt wird, ein oder mehrere vorgelatinisierte, quervernetzte Stärkederivate in einer Konzentration von 0,1 bis 20 Gewichts-%, besonders bevorzugt in einer Konzentration von 0,3 bis 15 Gewichts-% und ganz besonders bevorzugt in einer Konzentration von 0,5 bis 10 Gewichts-% bezogen auf das Gesamtgewicht der kosmetischen Zubereitung in der Zusammensetzung, mit welcher diese bei der Tränkung auf das Substrat aufgebracht wird, enthält.

Es ist erfindungsgemäß vorteilhaft, wenn als vorgelatinisierte, quervernetzte Stärkederivate hydroxypropylierte Phosphatester eingesetzt werden. Insbesondere vorteilhaft sind solche Stärkederivate, wie sie in der US 6248338 beschrieben werden, besonders vorteilhaft Hydroxypropyldistärkephosphat. Ganz besonders bevorzugt ist dabei der Einsatz eines Hydroxypropyldistärkephosphates, wie es als Produkt Structure® XL der Firma National Starch verkauft wird.

Die erfindungsgemäße kosmetische Zubereitung kann erfindungsgemäß vorteilhaft weitere kosmetische und/oder dermatologische Wirk-, Hilfs- und/oder Zusatzstoffe enthalten. Die in dieser Beschreibung angegebenen Mengenangaben beziehen sich dabei auf die Konzentrationen, wie sie in der Zubereitung vorliegen, bevor diese auf das Tuch aufgetragen wird.

Vorteilhafte waschaktive anionische Tenside im Sinne der vorliegenden Erfindung sind beispilsweise
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,
   Sulfonsäuren und deren Salze, wie
■ Acyl-isethionate, z.B. Natrium-/ Ammoniumcocoyl-isethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
   sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quarternäre Tenside. Quatemäre Tenside enthalten mindestens ein N-Atom, das mit 4 Alkyl- oder Arylgruppen kovalent verbunden ist. Vorteilhaft sind Benzalkoniumchlorid, Alkylbetain, Alkylamidopropylbetain und Alkyl-amidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.
   Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Es ist vorteilhaft im Sinn der vorliegenden Erfindung, wenn der Gehalt an einem oder mehreren waschaktiven Tensiden in der kosmetischen Zubereitung aus dem Bereich von 0,1 bis 25 Gew.-%, ganz besonders vorteilhaft von 10 bis 20 Gew.-% gewählt wird, jeweils bezogen auf das Gesamtgewicht der Zubereitungen.

Ferner können Polysorbate als waschaktive Agentien erfindungsgemäß vorteilhaft in die Emulsion eingearbeitet werden.

Die erfindungsgemäße kosmetische Zubereitung kann bevorzugt neben einer oder mehrerer Wasserphasen zusätzlich eine oder mehrere Ölphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion) sein. Die Art der Emulsion ("Emoulsionstyp") läßt sich mit den dem Fachmann hinlänglich bekannten Emulgatoren bzw. Emulgatormischungen nach Belieben einstellen.

Die erfindungsgemäße Zubereitung kann als wässrige Phase der Emulsion neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Die Ölphase der kosmetischen oder dermatologischen Reinigungsemulsionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölphase wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 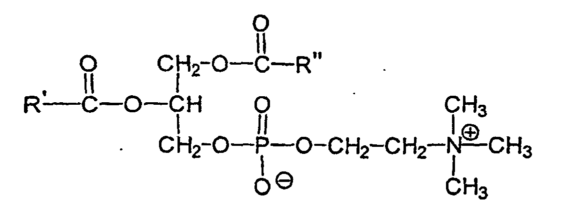 auszeichnen, wobei R' und R" typischerweise unverzweigte allphatische Reste mit 15 oder 17 Kohlenstoffatomen und bis zu 4 cis-Doppelbindungen darstellen.

Es ist erfindungsgemäß von Vorteil, wenn die kosmetische Reinigungsemulsion ein oder mehrere Polyacrylate enthält.

Erfindungsgemäß vorteilhafte Polyacrylate sind Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Polyacrylate sind Verbindungen der allgemeinen Strukturformel 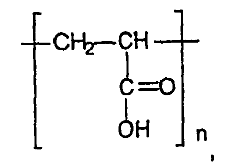 deren Molgewicht zwischen ca. 400 000 und mehr als 4 000 000 betragen kann. In die Gruppe der Polyacrylate gehören ferner Acrylat-Alkylacrylat-Copolymere, beispielsweise solche, die sich durch die folgende Struktur auszeichnen:

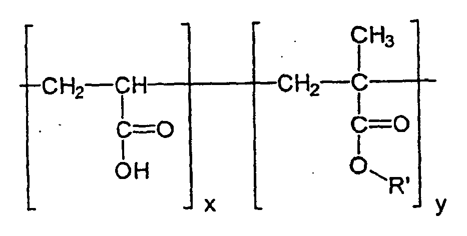

Darin stellen R' einen langkettigen Alkylrest und x und y Zahlen dar, welche den jeweiligen stöchiometrischen Anteil der jeweiligen Comonomere symbolisieren. Auch diese Polyacrylate sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984 oder auch die Typen ETD (Easy-to-disperse) 2001, 2020, 2050, wobei diese Verbindungen einzeln oder in beliebigen Kombinationen untereinander vorliegen können.

Ferner vorteilhaft im Sinne der vorliegenden Erfindung sind die den Acrylat-Alkylacrylat-Copolymeren vergleichbaren Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Es ist erfindungsgemäß von besonderem Vorteil, wenn als Polyacrylate C₁₀ bis C₃₀₋Alkylacrylat Copolymere eingesetzt werden.

Es ist vorteilhaft im Sinn der vorliegenden Erfindung, wenn der Gehalt an einem oder mehreren Polyacrylaten in der kosmetischen oder dermatologischen Reinigungsemulsion aus dem Bereich von 0,5 bis 2 Gew.-%, ganz besonders vorteilhaft von 0,7 bis 1,5 Gew.-% gewählt wird, jeweils bezogen auf das Gesamtgewicht der Zubereitungen

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, Emulgatoren, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, Antitranspirantien, Bleichmittel, Selbstbräuner, Repellentien, Filmbildner, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 10 Gew.-%, besonders bevorzugt 0,05 - 7 Gew.-%, insbesondere 0,1 - 5 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 5 Gew.-%, bezogen auf das Gesamtgewicht der Formulierung, zu wählen.

Darüber hinaus eignen sich ausgewählte erfindungsgemäße Rezepturen, welche z. B. bekannte Antifaltenwirkstoffe wie Flavonglycöside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung (z.B. Falten und Fältchen) auftreten. Weiterhin vorteilhaft eignen sie sich gegen das Erscheinungsbild der trockenen bzw. rauhen Haut.

Sofern α-Glycosylrutin das Antioxidants darstellt, ist vorteilhaft, deren jeweilige Konzentrationen aus dem Bereich von 0,001 - 5 Gew.-%, bezogen auf das Gesamtgewicht der Formulierung, zu wählen.

Auch ein Gehalt an UV-Lichtschutzfiltem ist vorteilhaft im Sinne der vorliegenden Erfindung, wobei alle nach der Positiv-Liste der Kosmetikverordnung (z.B. Anlage 7 der Kosmetikverordnung) zugelassenden organischen und anorganischen (Mikropigmente) UV-Lichtschutzfilter in beliebigen Abmischungen und Mengen in die Zubereitung einarbeitbar sind. Als anorganische Mikropigmente eignen sich beispielsweise die Oxyde des Titans, Zinks, Eisens, Zirkoniums, Siliciums, Mangans, Aluminiums und Cers sowie das Sulfat des Bariums. Diese Mikropigmente können auch nach den herkömmlichen Verfahren oberflächenbeschichtet sein.

Erfindungsgemäß vorteilhafte organische UV-Lichtschutzfilter stellen beispielsweise die Derivate der Zimtsäure, Salicylsäure, Aminobenzoesäure, der Benzoazolyle, Benzodiazolyle, Benzotriazole, Benzophenone, Benzimidazole (hier insbesondere die Sulfonsäure-derivate und Salze), Triazine sowie Trisiloxane, Phenylacrylate, und Kampferderivate dar.

Die erfindungsgemäß vorteilhaften einsetzbaren Wirk-, Hilfs- und Zusatzstoffe sind dabei keineswegs auf die hier namentlich erwähnten Stoffe und Verbindungen beschränkt.

Erfindungsgemäß ist auch das Verfahren zur Herstellung eines wasserunlöslichen Substrates, getränkt mit einer kosmetischen Zubereitung welches dadurch gekennzeichnet, dass die kosmetische Zubereitung auf das Substrat gesprüht oder das Substrat in ein Bad enthaltend die kosmetische Zubereitung getaucht wird.

Ferner ist das Verfahren zur Herstellung eines wasserunlöslichen Substrates, getränkt mit einer kosmetischen Zubereitung, dadurch gekennzeichnet, dass das Substrat im Anschluss an die Tränkung mit der kosmetischen Zubereitung getrocknet wird, erfindungsgemäß.

Nicht zuletzt ist die Verwendung des wasserunlöslichen Substrates, getränkt mit einer kosmetischen Zubereitung als trocken oder feucht anmutendes Reinigungs- oder Pflegetuch erfindungsgemäß. Insbesondere eignet sich das erfindungsgemäße wasserunlösliche Substrat zur Gesichtsreinigung und Babypflege.

Die folgenden Beispiele, in welchen die Zusammensetzung der erfindungsgemäßen kosmetischen Zubereitungen beschrieben werden, sollen die Erfindung erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

### Beispiele

### a) Beispielrezepturen für trocken anmutende, wasserunlösliche Substrate

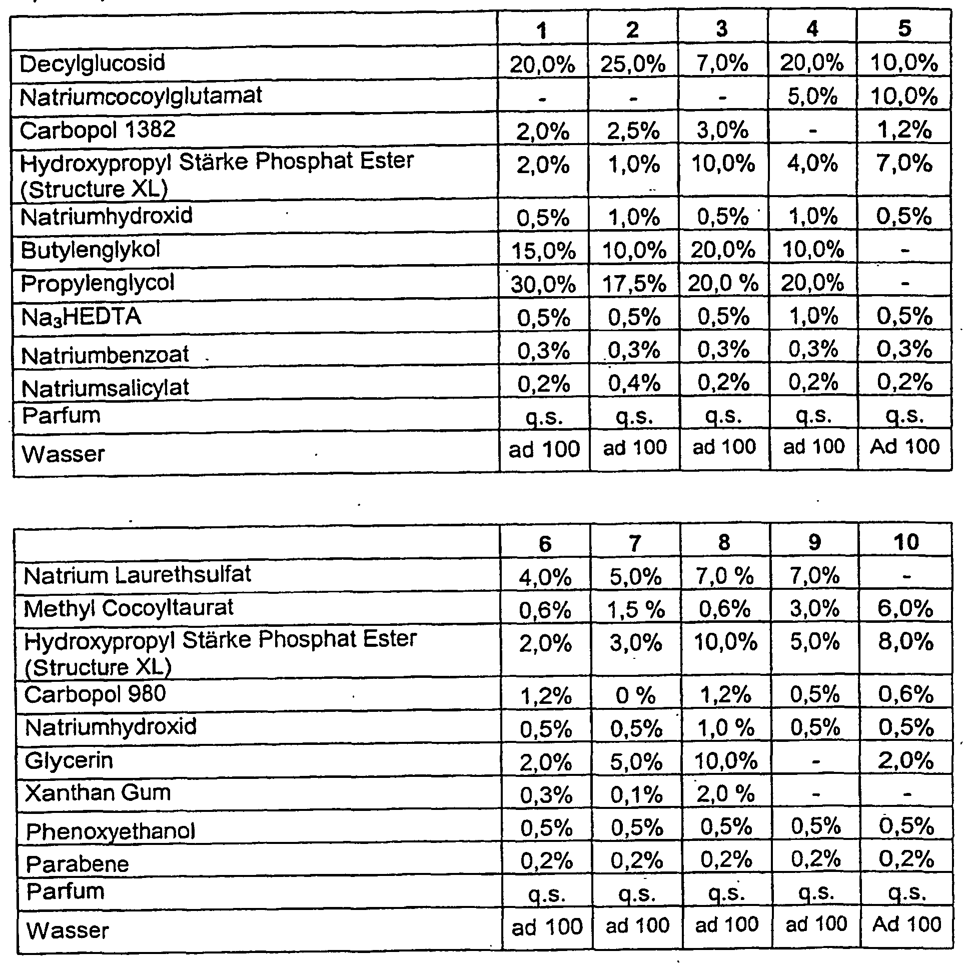

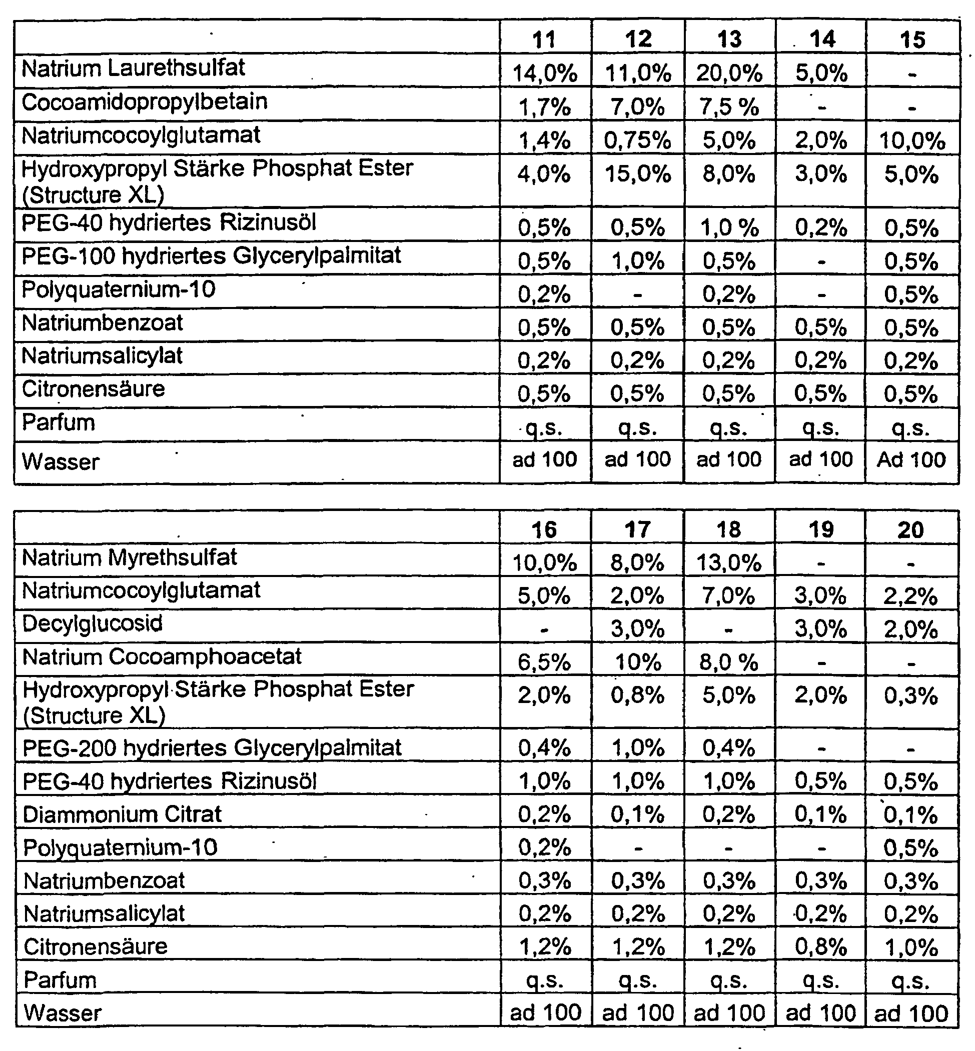

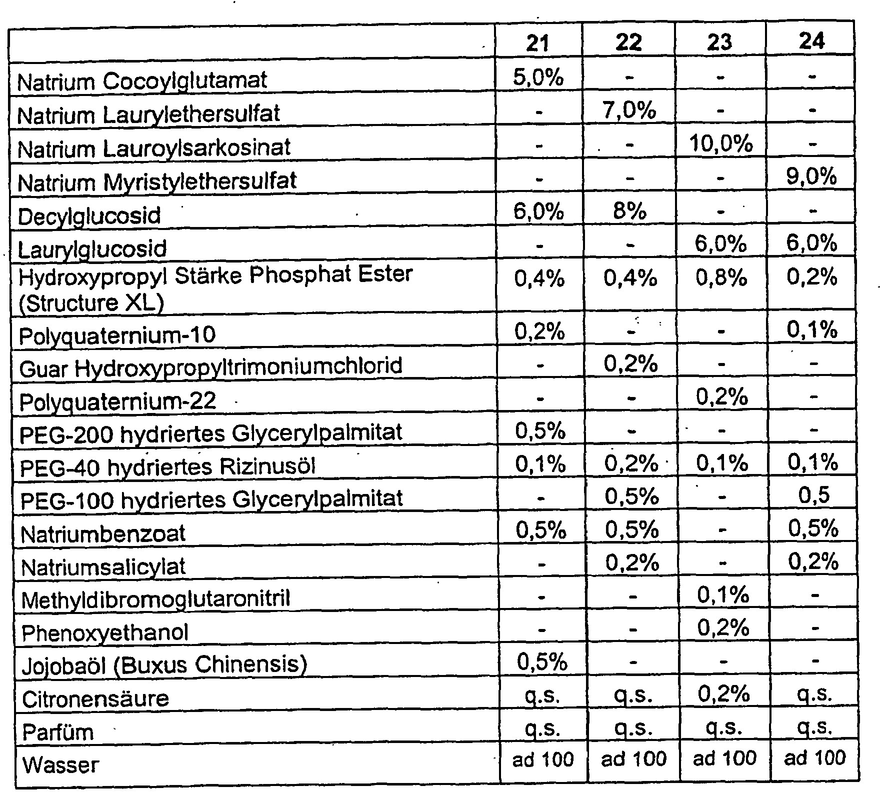

### Beispiel 25: Emulsion

| **Bestandteil** | **Menge / Gew.-%** | **Menge / Gew.-%** |
|---|---|---|
| Hydroxypropyl Stärke Phosphat Ester (Structure XL) | 5,0 | 10,0 |
| Paraffinum Liquidum | 1,0 | 8,0 |
| Glycerin | 14,0 | 5,0 |
| Octylstearat | 2,0 | 2,0 |
| Glyceryl Stearate, Ceteareth-20, Ceteareth-12, Cetearyl Alcohol, Cetyl Palmitate | 6,0 | 1,5 |
| Phenoxyeheanol, Methylparaben, Ethylparaben, Propylparaben, Butylparaben, Isobutylparaben | 0,5 | 0,5 |
| Parfum | q.s. | q.s. |
| Ceteareth-20 | 20,0 | 0,3 |
| Methylparaben | 1,0 | 0,3 |
| Wasser | ad 100,0 | ad 100,0 |

### Beispiel 26: wäßrige Tränkungslösung

| **Bestandteil** | **Menge / Gew.-%** | **Menge / Gew.-%** |
|---|---|---|
| Hydroxypropyl Stärke Phosphat Ester (Structure XL) | 10,0 | 20,0 |
| Butylenglykol | 1,0 | 1,0 |
| PEG-40 Hydrogenated Castor Oil | 1,0 | 0,8 |
| Phenoxyehanol, Methylparaben, Ethylparaben, Propylparaben, Butylparaben, Isobutylparaben | 1,5 | 0,7 |
| Kaliumsorbat | 0,5 | 0,3 |
| Parfum | q.s. | q.s. |
| Zitronensäure | 0,5 | 0,2 |
| Wasser | ad 100,0 | ad 100,0 |

### Beispiel 27: alkoholische Tränkungslösung

| **Bestandteil** | **Menge / Gew.-%** | **Menge / Gew.-%** |
|---|---|---|
| Hydroxypropyl Stärke Phosphat Ester (Structure XL) | 8,0 | 15,0 |
| Ethanol | 60,0 | 60,0 |
| Glycerin | 5,0 | 15,0 |
| Isopropylalkohol | - | 5,0 |
| Ethylenediamine | - | 1,0 |
| Dexpanthenol | - | 1,0 |
| Carbomer | - | 3,0 |
| Parfum | q.s. | q.s. |
| Farbstoff | 0 | 0,5 |
| Wasser | ad 100,0 | ad 100,0 |

### b) Beispielrezepturen für feucht anmutende, wasserunlösliche Substrate

### Beispiel 1:

| **Bestandteil** | **Menge / Gew.-%** |
|---|---|
| Hydroxypropyl Stärke Phosphat Ester (Structure XL) | 0,5 |
| Parfum | q.s. |
| Paraffinum Liquidum | ad 100,0 |

### Beispiel 2: Emulsion

| **Bestandteil** | **Menge / Gew.-%** | **Menge / Gew.-%** |
|---|---|---|
| Hydroxypropyl Stärke Phosphat Ester (Structure XL) | 8,0 | 3,0 |
| Paraffinum Liquidum | 0,5 | 8,0 |
| Glycerin | 7,0 | 5,0 |
| Octylstearat | 1,0 | 2,0 |
| Glyceryl Stearate, Ceteareth-20, Ceteareth-12, Cetearyl Alcohol, Cetyl Palmitate | 3,0 | 1,5 |
| Phenoxyeheanol, Methylparaben, Ethylparaben, Propylparaben, Butylparaben, Isobutylparaben | 0,5 | 0,5 |
| Parfum | q.s. | q.s. |
| Ceteareth-20 | 10,0 | 0,3 |
| Methylparaben | 1,0 | 0,3 |
| Wasser | ad 100,0 | ad 100,0 |

### Beispiel 4: wäßrige Tränkungslösung

| **Bestandteil** | **Menge / Gew.-%** | **Menge / Gew.-%** |
|---|---|---|
| Hydroxypropyl Stärke Phosphat Ester (Structure XL) | 10,0 | 7,0 |
| Butylenglykol | 1,0 | 1,0 |
| PEG-40 Hydrogenated Castor Oil | 1,0 | 0,8 |
| Phenoxyehanol, Methylparaben, Ethylparaben, Propylparaben, Butylparaben, Isobutylparaben | 1,5 | 0,7 |
| Kaliumsorbat | 0,5 | 0,3 |
| Parfum | q.s. | q.s. |
| Zitronensäure | 0,5 | 0,16 |
| Wasser | ad 100,0 | ad 100,0 |

### Beispiel 6

| **Bestandteil** | **Menge / Gew.-%** |
|---|---|
| Hydroxypropyl Stärke Phosphat Ester (Structure XL) | 0,5 |
| Dimethicone | 20,0 |
| Silikongum | 7,0 |
| Phenyltrimethylmethicone | 7,0 |
| Parfum | q.s. |
| Cyclomethicone | ad 100,0 |

### Beispiel 8: alkoholische Tränkungslösung

| **Bestandteil** | **Menge / Gew.-%** | **Menge / Gew.-%** |
|---|---|---|
| Hydroxypropyl Stärke Phosphat Ester (Structure XL) | 2,0 | 8,0 |
| Ethanol | 60,0 | 60,0 |
| Glycerin | 5,0 | 5,0 |
| Isopropylalkohol | 0 | 5,0 |
| Ethylenediamine | 0 | 1,0 |
| Dexpanthenol | 0 | 1,0 |
| Carbomer | 0 | 3,0 |
| Parfum | q.s. | q.s. |
| Farbstoff | 0 | 0,5 |
| Wasser | ad 100,0 | ad 100,0 |

### Beispiel 9: After Sun-/Hautpflege-Emulsion

| **Bestandteil** | **Menge / Gew.-%** |
|---|---|
| Hydroxypropyl Stärke Phosphat Ester (Structure XL) | 5,0 |
| Ceteth-15 | 6,0 |
| Glycerylisostearate | 2,0 |
| Cetyl Alkohol | 1,0 |
| Dicaprylyl Carbonate | 5,0 |
| Octyldodecanol | 3,0 |
| Cylomethicone | 1,0 |
| Butylene Glycol | 3,0 |
| Ethanol | 5,0 |
| DMDM Hydantoin | 0,6 |
| Octoxyglycerin | 1,0 |
| Antioxydantien | 0,5 |
| Parfuem | q.s. |
| Farbstoffe | 0,3 |
| Wasser | ad 100 |

### Beispiel 10: nicht fettende Körperpflegeemulsion

| **Bestandteil** | **Menge / Gew.-%** |
|---|---|
| Hydroxypropyl Stärke Phosphat Ester (Structure XL) | 4,0 |
| Ceteareth-12 | 6,0 |
| Glyceryl Stearate | 3,5 |
| Cetyl Palmitate | 3,0 |
| Dicaprylyl Ether | 5,0 |
| Cycolmethicone | 3,0 |
| Phenyl Trimethicone | 1,0 |
| Paraffinwax | 2,0 |
| Glycerin | 7,5 |
| Parabene | 1,0 |
| Phenoxyethanol | 1,0 |
| AGR | 0,5 |
| Parfuem | q.s. |
| Farbstoffe | 0,5 |
| Wasser | ad 100 |

### Beispiel 11: Sonnenschutzmittel für seidiges Hautgefühl

| **Bestandteil** | **Menge / Gew.-%** | **Menge / Gew.-%** |
|---|---|---|
| Hydroxypropyl Stärke Phosphat Ester (Structure XL) | 3,6 | 6,0 |
| Ceteareth-20 | 5,5 | 6,5 |
| Glyceryl Stearate | 4,0 | 2,0 |
| Stearyl Alkohol | 3,0 | 1,0 |
| Dicaprylyl Ether | 5,0 | 5,0 |
| Octyldodecanol | 3,0 | 3,0 |
| Phenyl Trimethicone | 1,0 | - |
| Bis-Ethylhexyloxyphenol Methoxyphenyl Triazine | 2,0 | - |
| Octocrylene | 7,0 | 7,0 |
| Diethylhexyl Butamido Triazone | 1,0 | - |
| Ethylhexyl Methoxycinnamate | 4,0 | 4,0 |
| Butylene Glycol | 1,0 | - |
| Vitamin E Acetat | 1,0 | - |
| C12-15 Alkyl Benzoate | - | 1,0 |
| Titandioxid | - | 2,0 |
| Bis-Ethylhexyloxyphenol Methoxyphenyl Triazine | - | 2,0 |
| PVP/Hexadecene Copolymer | 1,0 | - |
| Parabene | 1,0 | 1,0 |
| Antioxydantien | 0,5 | 0,5 |
| Parfuem | q.s. | q.s. |
| Wasser | ad 100 | ad 100 |

### Beispiel 13: Sonnenschutzformulierung

| **Bestandteil** | **Menge / Gew.-%** |
|---|---|
| Hydroxypropyl Stärke Phosphat Ester (Structure XL) | 5,0 |
| Steareth-20 | 6,5 |
| Glycerylisostearate | 2,0 |
| Cetyl Alkohol | 1,0 |
| Dicaprylyl Carbonate | 5,0 |
| Shea Butter | 3,0 |
| C12-15 Alkyl Benzoate | 1,0 |
| Bis-Ethylhexyloxyphenol Methoxyphenyl Triazine | 2,0 |
| Butylmethoxydibenzoylmethane | 1,0 |
| Ethylhexyl Triazone | 2,0 |
| Phenylbenzimidazol Sulfonsäure | 2,0 |
| Ethylhexyl Methoxycinnamate | 4,0 |
| Glycerin | 10,0 |
| Tricontanyl PVP | 1,0 |
| Citrat-Puffer | 1,0 |
| Parabene | 1,0 |
| Antioxydantien | 0,5 |
| Parfuem | q.s. |
| Wasser | ad 100 |

### Beispiel 14: Sonnenschutzformulierung

| **Bestandteil** | **Menge / Gew.-%** |
|---|---|
| Hydroxypropyl Stärke Phosphat Ester (Structure XL) | 3,0 |
| Ceteareth-30 | 7,0 |
| Glycerylisostearate | 2,5 |
| Cetyl Alkohol | 1,0 |
| Dicaprylyl Carbonate | 4,0 |
| Capric/Caprylic Triglyceride | 2,0 |
| C12-15 Alkyl Benzoate | 6,0 |
| Methylen Bis-Benzotriazolyl Tetramethylbutylphenol | 2,0 |
| Butyl Methoxydibenzoylmethane | 2,0 |
| Ethylhexyl Triazone | 4,0 |
| Bis-Imidazylat | 2,0 |
| Methylbenzylidene Camphor | 4,0 |
| Glycerin | 5,0 |
| PVP Hexadecene Copolymer | 1,0 |
| Parabene | 1,0 |
| Antioxydantien | 0,5 |
| Parfuem | q.s. |
| Wasser | ad 100 |

### Beispiel 15: Sonnenschutzformulierung

| **Bestandteil** | **Menge / Gew.-%** |
|---|---|
| Hydroxypropyl Stärke Phosphat Ester (Structure XL) | 4,0 |
| Ceteareth-20 | 7,5 |
| Glyceryl Stearate | 3,0 |
| Cetyl Palmitate | 1,5 |
| Dicaprylyl Carbonate | 5,0 |
| Cocoglycerides | 2,0 |
| C12-15 Alkyl Benzoate | 6,0 |
| Barium Sulfate | 2,0 |
| Bis-Ethylhexyloxyphenol Methoxyphenyl Triazine | 2,0 |
| Ethylhexyl Triazone | 4,0 |
| Bis-Imidazylat | 1,0 |
| Phenylbenzimidazol Sulfonsäure | 2,0 |
| Methylbenzylidene Camphor. | 4,0 |
| PVP Hexadecene Copolymer | 1,0 |
| NaOH | 0,5 |
| Parabene | 1,0 |
| Antioxydantien | 0,5 |
| Parfuem | q.s. |
| Wasser | ad 100 |

### Beispiel 16: After Sun-/Hautpflege-Formulierung

| **Bestandteil** | **Menge / Gew.-%** |
|---|---|
| Hydroxypropyl Stärke Phosphat Ester (Structure XL) | 10,0 |
| Ceteth-15 | 6,0 |
| Glycerylisostearate | 2,0 |
| Cetyl Alkohol | 1,0 |
| Dicaprylyl Carbonate | 5,0 |
| Shea Butter | 1,0 |
| Octyldodecanol | 3,0 |
| Cylomethicone | 1,0 |
| Mineral Oil | 2,0 |
| Ethanol | 5,0 |
| Parabene | 1,0 |
| Antioxydantien | 0,5 |
| Parfuem | q.s. |
| Wasser | ad 100 |

### Öl ― Beispiele

### Öl-1

| **Bestandteil** | **Menge / Gew.-%** |
|---|---|
| Hydroxypropyl Stärke Phosphat Ester (Structure XL) | 0,5 |
| Capric/Caprylic Triglyceride | 2,0 |
| C12-15 Alkyl Benzoate | 6,0 |
| Butyl Methoxydibenzoylmethane | 2,0 |
| Ethylhexyl Triazone | 2,0 |
| Bis-Ethylhexyloxyphenol Methoxyphenyl Triazine | 1,0 |
| Methylbenzylidene Camphor | 4,0 |
| Shea Butter | 1,0 |
| Butylene Glycol Dicaprate/Dicaprylate | 3,0 |
| Dimethicone | 5,0 |
| Parabene | 1,0 |
| Antioxydantien | 0,5 |
| Parfuem | q.s. |
| Mineraloil | ad 100 |

### Öl-2

| **Bestandteil** | **Menge / Gew.-%** | **Menge / Gew.-%** | **Menge / Gew.-%** |
|---|---|---|---|
| Hydroxypropyl Stärke Phosphat Ester (Structure XL) | 0,5 | 0,5 | 1,0 |
| Dicaprylyl Carbonate | 5,0 | 5,0 | 5,0 |
| Bis-Ethylhexyloxyphenol Methoxyphenyl Triazine | 2,0 | 2,0 | - |
| Ethylhexyl Triazone | 4,0 | - | - |
| Methylbenzylidene Camphor | 4,0 | - | - |
| Shea Butter | 1,0 | 1,0 | 1,0 |
| Octyldodecanol | 3,0 | - | - |
| Cylomethicone | 1,0 | - | - |
| Diethylhexyl Butamido Triazone | - | 4,0 | - |
| Methylbenzylidene Camphor | - | 1,0 | - |
| Ethylhexyl Methoxycinnamate | - | - | 10,0 |
| Bis-Ethylhexyloxyphenol Methoxyphenyl Triazine | - | - | 2,0 |
| Diethylhexyl Butamido Triazone | - | - | 4,0 |
| Octocrylene | - | - | 5,0 |
| Phenyltrimethicone | - | 1,0 | 1,0 |
| Vitamin E | 1,0 | 2,0 | 1,0 |
| Parfuem | q.s. | q.s. | q.s. |
| Mineraloil | ad 100 | ad 100 | ad 100 |

### Wäßrige Formulierung:

| **Bestandteil** | **Menge / Gew.-%** |
|---|---|
| Hydroxypropyl Stärke Phosphat Ester (Structure XL) | 10,0 |
| Bis-Imidazylat | 1,0 |
| Phenylbenzimidazol Sulfonsäure | 2,0 |
| Glycerin | 10,0 |
| Parabene | 1,0 |
| Antioxydantien | 0,5 |
| Parfuem | q.s. |
| Wasser | ad 100 |

### Tensidrezepturbeispiele (Menge / Gew.-%):

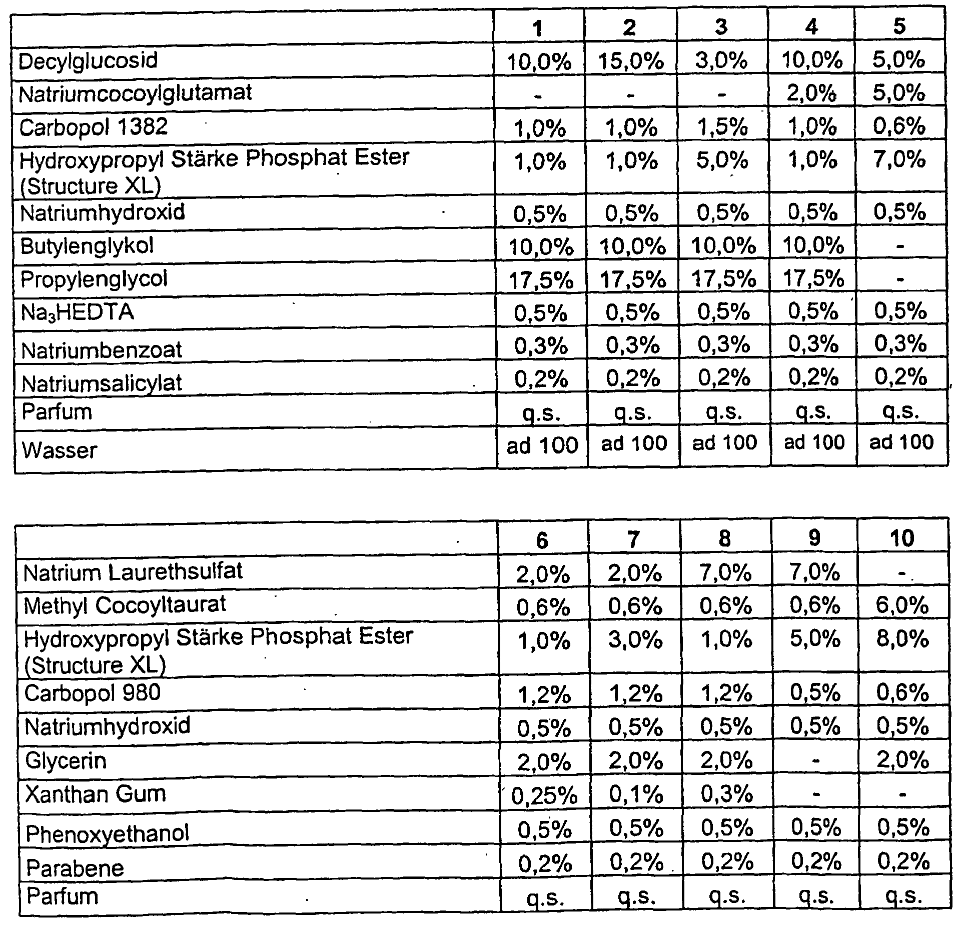

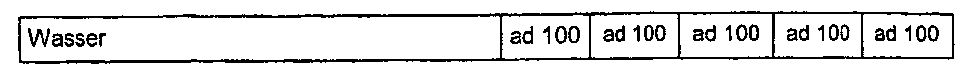

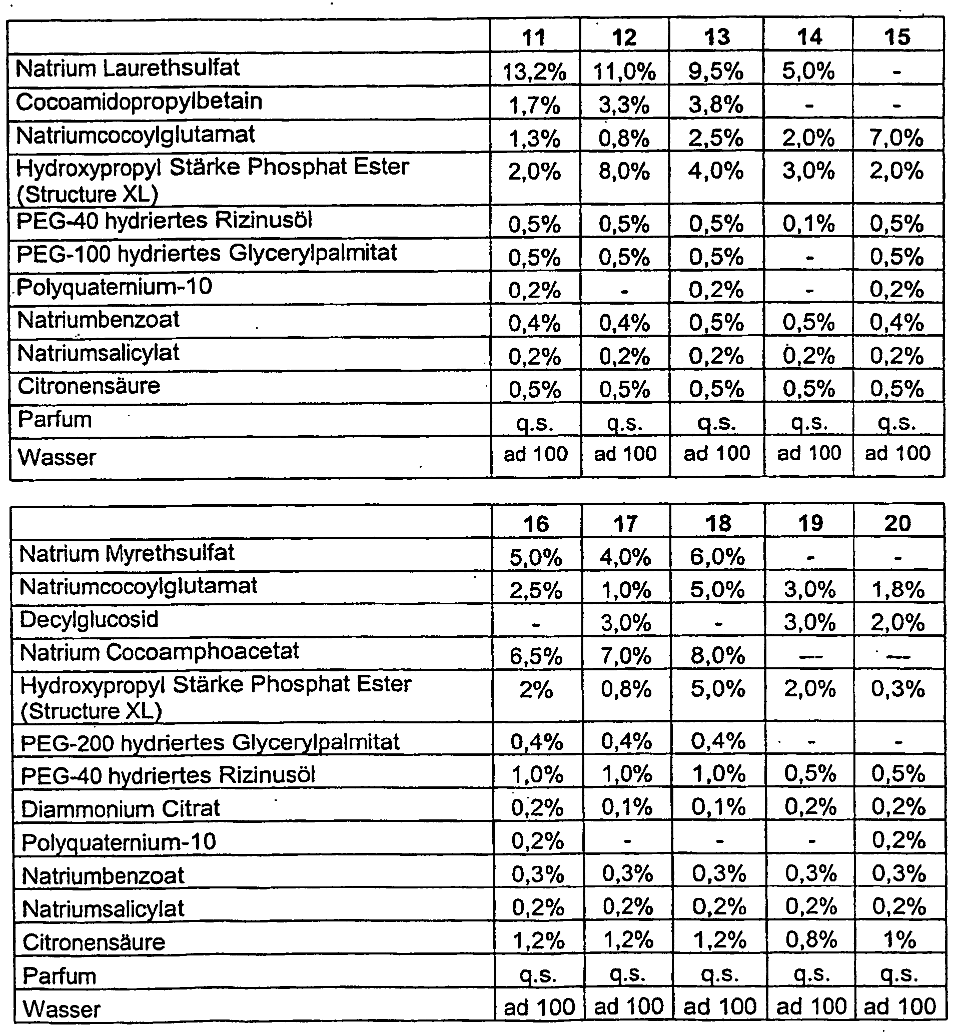

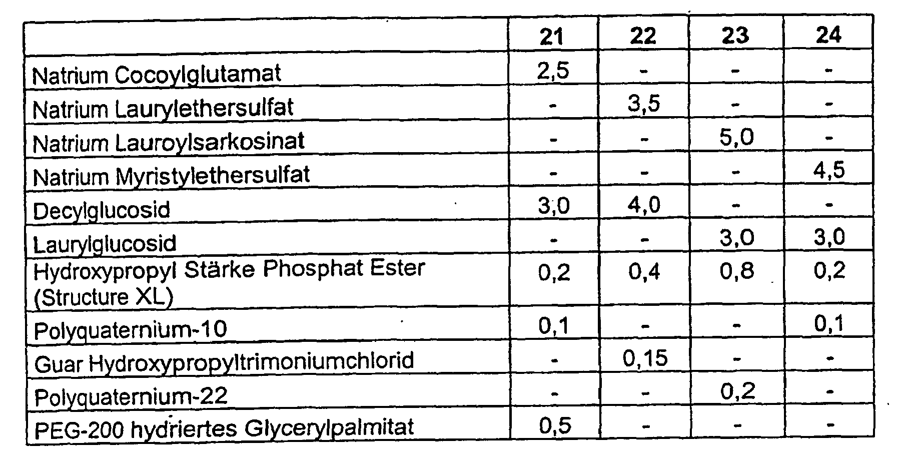

## Patentansprüche

1. Wasserunlösliches Substrat, imprägniert mit einer kosmetischen Zubereitung enthaltend ein oder mehrere vorgelatinisierte, quervernetzte Stärkederivate.

2. Wasserunlösliches Substrat nach Anspruch 1, **dadurch gekennzeichnet, dass** als wasserunlösliches Substrat ein Vlies verwendet wird.

3. Wasserunlösliches Substrat nach einem der Ansprüche 1 oder 2, **dadurch gekennzeichnet, dass** als wasserunlösliches Substrat ein Tuch verwendet wird.

4. Wasserunlösliches Substrat nach einem der Ansprüche 1 bis 3, **dadurch gekennzeichnet, dass** die kosmetische Zubereitung ein oder mehrere vorgelatinisierte, quervernetzte Stärkederivate in einer Konzentration von 0,1 bis 20 Gewichts-%, bezogen auf das Gesamtgewicht der kosmetischen Zubereitung in der Zusammensetzung, mit welcher diese bei der Tränkung auf das Substrat aufgebracht wird, enthält.

5. Wasserunlösliches Substrat nach einem der Ansprüche 1 bis 4, **dadurch gekennzeichnet, dass** als vorgelatinisierte, quervernetzte Stärkederivate hydroxypropylierte Phosphatester eingesetzt werden.

6. Wasserunlösliches Substrat nach einem der Ansprüche 1 bis 5, **dadurch gekennzeichnet, dass** als vorgelatinisiertes, quervernetztes Stärkederivat Hydroxypropyldistärkephosphat (CAS Nummer 113894-92-1) eingesetzt wird.

7. Wasserunlösliches Substrat nach einem der Ansprüche 1 bis 5, **dadurch gekennzeichnet, dass** die kosmetische Zubereitung weitere kosmetische und/oder dermatologische Wirk-, Hilfs- und/oder Zusatzstoffe enthält.

8. Verfahren zur Herstellung eines wasserunlöslichen Substrates nach einem der Ansprüche 1 bis 7, **dadurch gekennzeichnet, dass** die kosmetische Zubereitung auf das Substrat gesprüht oder das Substrat in ein Bad enthaltend die kosmetische Zubereitung getaucht wird.

9. Verfahren nach Anspruch 8 zur Herstellung eines wasserunlöslichen Substrates, **dadurch gekennzeichnet, dass** das Substrat im Anschluss an die Tränkung mit der kosmetischen Zubereitung getrocknet wird.

10. Verwendung des wasserunlöslichen Substrates nach einem der Ansprüche 1 bis 7 als trocken oder feucht anmutendes Reinigungs- oder Pflegetuch.

## Revendications

1. Support insoluble dans l'eau, imprégné avec une préparation cosmétique contenant un ou plusieurs dérivés d'amidon transversalement réticulés, prégélatinisés.

2. Support insoluble dans l'eau selon la revendication 1, **caractérisé en ce qu'**un non-tissé est utilisé en tant que support insoluble dans l'eau.

3. Support insoluble dans l'eau selon la revendication 1 ou 2, **caractérisé en ce qu'**une lingette est utilisée en tant que support insoluble dans l'eau.

4. Support insoluble dans l'eau selon l'une quelconque des revendications 1 à 3, **caractérisé en ce que** la préparation cosmétique contient un ou plusieurs dérivés d'amidon transversalement réticulés, prégélatinisés, à une concentration de 0,1 à 20 % en poids, par rapport au poids total de la préparation cosmétique dans la composition avec laquelle celle-ci est appliquée lors de l'imprégnation sur le support.

5. Support insoluble dans l'eau selon l'une quelconque des revendications 1 à 4, **caractérisé en ce que** des esters phosphoriques hydroxypropylés sont utilisés en tant que dérivés d'amidon transversalement réticulés, prégélatinisés.

6. Support insoluble dans l'eau selon l'une quelconque des revendications 1 à 5, **caractérisé en ce que** du phosphate d'hydroxypropyldiamidon (numéro CAS 113894-92-1) est utilisé en tant que dérivé d'amidon transversalement réticulé, prégélatinisé.

7. Support insoluble dans l'eau selon l'une quelconque des revendications 1 à 5, **caractérisé en ce que** la préparation cosmétique contient d'autres actifs, adjuvants et/ou additifs cosmétiques et/ou dermatologiques.

8. Procédé pour la production d'un support insoluble dans l'eau selon l'une quelconque des revendications 1 à 7, **caractérisé en ce que** la préparation cosmétique est pulvérisée sur le support ou le support est plongé dans un bain contenant la préparation cosmétique.

9. Procédé pour la production d'un support insoluble dans l'eau selon la revendication 8, **caractérisé en ce qu'**à la suite de l'imprégnation avec la préparation cosmétique, le support est séché.

10. Utilisation du support insoluble dans l'eau selon l'une quelconque des revendications 1 à 7, en tant que lingette de soin ou de nettoyage donnant une impression sèche ou humide.

## Claims

1. Water-insoluble substrate impregnated with a cosmetic preparation comprising one or more pregelatinized crosslinked starch derivatives.

2. Water-insoluble substrate according to Claim 1, **characterized in that** the water-insoluble substrate used is a nonwoven.

3. Water-insoluble substrate according to one of Claims 1 or 2, **characterized in that** the water-insoluble substrate used is a tissue.

4. Water-insoluble substrate according to one of Claims 1 to 3, **characterized in that** the cosmetic preparation comprises one or more pregelatinized crosslinked starch derivatives in a concentration of from 0.1 to 20% by weight, based on the total weight of the cosmetic preparation in the composition, with which the latter is applied to the substrate during impregnation.

5. Water-insoluble substrate according to one of Claims 1 to 4, **characterized in that** the pregelatinized crosslinked starch derivatives used are hydroxypropylated phosphate esters.

6. Water-insoluble substrate according to one of Claims 1 to 5, **characterized in that** the pregelatinized crosslinked starch derivative used is hydroxypropyl distarch phosphate (CAS number 113894-92-1).

7. Water-insoluble substrate according to one of Claims 1 to 5, **characterized in that** the cosmetic preparation comprises further cosmetic and/or dermatological active ingredients, auxiliaries and/or additives.

8. Process for the preparation of a water-insoluble substrate according to one of Claims 1 to 7, **characterized in that** the cosmetic preparation is sprayed onto the substrate or the substrate is dipped into a bath containing the cosmetic preparation.

9. Process according to Claim 8 for the preparation of a water-insoluble substrate, **characterized in that** the substrate is dried after being impregnated with the cosmetic preparation.

10. The use of the water-insoluble substrate according to one of Claims 1 to 7 as a cleansing or care tissue which appears to be dry or wet.
